# EUROPÄISCHE PATENTSCHRIFT

(11) **EP 0 913 690 B1**
(45) Veröffentlichungstag und Bekanntmachung des Hinweises auf die Patenterteilung: **21.01.2009**
(21) Anmeldenummer: 98120756.6
(22) Anmeldetag: 02.11.1998
(51) Int. Cl.: G01N 33/48, G01N 33/543

(54) **Polyethylenglykol-derivatisierte Biomoleküle und deren Verwendung in heterogenen Nachweisverfahren**
Polyethylene glycol derivatised biomolecules and their use in hetergeneous detection methods
Biomolecules dérivées du polyethyleneglycol et leurs utilisations dans les méthodes de détection hétérogène

(30) Priorität: 03.11.1997 DE 19748489
(43) Veröffentlichungstag der Anmeldung: 06.05.1999
(60) Teilanmeldung: 08017911.2
(73) Patentinhaber: Roche Diagnostics GmbH, 68305 Mannheim (DE)
(72) Erfinder: Hornauer, Hans, Dr., 82380 Peissenberg (DE); Lenz, Helmut, Dr., 82327 Tutzing (DE); Sluka, Peter, Dr., 82362 Weilheim (DE); Karl, Johann, Dr., 82380 Peissenberg (DE); Mutter, Wolfgang, Dr., 82347 Bernried (DE)
(74) Vertreter: Weiss, Wolfgang

(56) Entgegenhaltungen:
- EP-A- 0 664 452
- EP-A- 0 713 095
- WO-A-94/27137
- DATABASE WPI Section Ch, Week 199612 Derwent Publications Ltd., London, GB; Class B04, AN 1996-112719 XP002193414 & JP 08 012699 A (GH KIRIKAGE GAKUEN), 16. Januar 1996 (1996-01-16)

## Beschreibung

Die Erfindung betrifft heterogene Verfahren zum Nachweis eines Analyten in einer Probe und für solche Verfahren geeignete Reagenzienkits.

Verfahren zum Nachweis eines Analyten in einer Probe, bei denen eine Festphase verwendet wird, werden als heterogene Testformate bezeichnet. Ein Problem bei derartigen Verfahren besteht darin, daß oftmals eine unspezifische Bindung von Bestandteilen der Probe oder von Testreagenzien an die Festphase auftritt, wodurch falsche Testergebnisse erhalten werden können. Besonders häufig werden diese unspezifischen Bindungen bei Proben aus Körperflüssigkeiten, z.B. Serum oder Plasma beobachtet. Um diese unspezifischen Bindungen zu unterdrücken, besteht die Möglichkeit, den Reagenzien oberflächenaktive Substanzen wie z.B. Tween 20 zuzusetzen (WO88/07683). Bekannt ist außerdem die Funktionalisierung von metallischen Oberflächen (Whitesides et al., Science 252 (1991), 1164-1166) und von oxidischen Oberflächen (EP-A-0 664 452) mit reaktiven Polyethylenglykol-Derivaten, um die unspezifische Bindung auf der Oberfläche zu minimieren.

Dem Stand der Technik zur Verringerung der unspezifischen Bindung von oberflächenaktiven Substanzen haben den Nachteil, daß sie an der Festphase gebundene Moleküle, z.B. Festphasenrezeptoren, verdrängen und auf diese Weise die Testfunktion beeinträchtigen. Darüber hinaus werden als oberflächenaktive Substanzen in der Regel großtechnisch hergestellte Waschmitteltenside verwendet, die in ihrer Zusammensetzung heterogen sind und gelegentlich Verunreinigungen enthalten. Die daraus resultierenden Chargenschwankungen führen oft zu Störungen und nicht reproduzierbaren Ergebnissen. Darüber hinaus können empfindliche Festphasenmoleküle, z.B. Proteine, durch die oberflächenaktiven Substanzen in ihrer Struktur gestört und letztlich denaturiert werden.

Die aus dem Stand der Technik bekannte Funktionalisierung von metallischen oder oxidischen Oberflächen mit Polyethylenglykol ist einerseits auf bestimmte Arten von Oberflächen beschränkt und andererseits nicht ausreichend, um die unspezifische Bindung an eine auf der Festphasenoberfläche aufgebrachte Schicht von Biomolekülen zu verhindern.

Die der vorliegenden Erfindung zugrundeliegende Aufgabe bestand somit darin, ein neues Verfahren zur Verringerung der unspezifischen Bindung an eine Festphase beim Nachweis eines Analyten in einer Probe bereitzustellen, bei der die Nachteile des Standes der Technik mindestens teilweise vermieden werden können.

Ein erster Aspekt der vorliegenden Erfindung betrifft ein heterogenes Verfahren zum Nachweis eines Analyten in einer Probe, umfassend die Schritte:
(a) Bereitstellen einer Festphase, umfassend in immobilisierter Form einen analytspezifischen Festphasenreaktanden und ein analytunspezifisches Biomolekül, das mit einem Poly (C₂-C₃)-Alkylenoxid gekoppelt ist, wobei die Festphase mit einem ersten Partner eines hochaffinen Bindepaares belegt ist, an die ein Konjugat des analytspezifischen Festphasenreaktanden mit dem zweiten Partner des Bindepaares immobilisiert ist und wobei als analytunspezifisches Biomolekül eine Blockierungssubstanz verwendet wird, die den zweiten Partner des Bindepaares umfaßt und ausgewählt ist aus Konjugaten der allgemeinen Strukturformel (Ia) oder (Ib):

   Pᵣ[- (AOₙ)T]ₘ (Ia)

   Pᵣ-I-[-(AOₙ)T]ₘ (Ib)

   worin
   - P: Biotin oder ein Biotinderivat ist,
   - l: ein analytunspezifisches Biomolekül ist,
   - r: eine Zahl von 1 bis 10 ist,
   - AO: eine (C₂-C₃)-Alkylenoxidgruppe ist,
   - n: eine Zahl von 5 bis 500 ist,
   - T: eine Endgruppe vorzugsweise ausgewählt aus OH, C₁-C₄-Alkoxy und C₁-C₄-Acyl ist und
   - m: eine Zahl von 1 bis 10 ist,
(b) Inkubieren der Probe mit der Festphase und einem Testreagenz und
(c) Nachweisen des Vorhandenseins oder/und der Menge des Analyten in der Probe.

Durch Blockierung der Festphase mit einem Polyalkylenoxid-, insbesondere mit einem Polyethylenglykol-modifizierten analytunspezifischen Biomolekül konnte eine deutliche Verringerung der unspezifischen Bindung von Probenbestandteilen an die Festphase erreicht werden, ohne daß gleichzeitig die Testsensitivität signifikant beeinträchtigt wurde. Die Zugabe des Blockierungsreagenz kann während oder/und nach der Immobilisierung des Festphasenreaktanden erfolgen. Besonders bevorzugt wird eine mit einem analytspezifischen Festphasenreaktanden vorbelegte Festphase nachträglich mit einem analytunspezifischen Biomolekül blockiert.

Gute Ergebnisse wurden bei Verwendung von Festphasen erhalten, die "begrenzte Testflächen" aufweisen, d.h. mit einem Festphasenreaktanden belegte begrenzte Bereiche, die durch inerte Bereiche von weiteren Testflächen räumlich getrennt sind. Besonders bevorzugt sind flächig mit einer analytunspezifischen Vorbeschichtung, z.B. mit Streptavidin, überzogene Festphasen, die mindestens eine räumlich begrenzte Testfläche, auf der ein analytspezifischer Festphasenraktand immobilisiert ist, enthalten. Die begrenzten Testflächen haben bevorzugt einen Durchmesser von 10 µm bis 10 mm. Besonders bevorzugt sind miniaturisierte Testflächen mit einem Durchmesser von 10 µm bis 2 mm. Weiterhin bevorzugt sind Festphasen mit mehreren Testflächen, die unterschiedliche analytspezifische Festphasenreaktanden enthalten können und auch als Array-Systeme bezeichnet werden (vgl. z.B. US-Patente 5,432,099; 5,516,635 und 5,126,276). Mit diesen Array-Systemen können mehrere Analytbestimmungen gleichzeitig an einer Probe durchgeführt werden.

Die Festphase im erfindungsgemäßen Verfahren umfaßt einen beliebigen Träger, wobei nichtporöse Träger, z.B. Träger mit Kunststoff-, Glas-, Metall- oder Metalloxidoberfläche bevorzugt sind. Auch poröse Träger, wie etwa Teststreifen sind geeignet.

Auf der Festphase wird ein analytspezifischer Festphasenreaktand immobilisiert, d.h. ein Biomolekül, welches eine spezifische Wechselwirkung mit einem zu bestimmenden Analyten oder - im Falle kompetitiver Testformate -mit einem Analogon eines zu bestimmenden Analyten eingehen kann. Beispiele für analytspezifische Festphasenreaktanden sind Antikörper, Antigene, Peptide, Haptene, Nukleinsäuren, Nukleinsäureanaloga, Glykoproteine, Saccharide, Lipoproteine und andere Biomoleküle.

Die Immobilisierung des Festphasenreaktanden erfolgt durch Kopplung über hochaffine Bindepaare. Hierzu wird die Festphase zunächst mit einem ersten Partner eines hochaffinen Bindepaares belegt und daran ein Konjugat des Festphasenreaktanden mit dem zweiten Partner des Bindepaares immobilisiert. Beispiele für geeignete hochaffine Bindepaare sind Streptavidin oder Avidin/Biotin oder ein Biotinderivat (beispielsweise Desthiobiotin, Iminobiotin, Aminobiotin oder eine andere mit hoher Affinität an Streptavidin oder Avidin bindefähige Substanz). Besonders bevorzugt verwendet man als hochaffines Bindepaar Streptavidin oder Avidin/Biotin.

Das erfindungsgemäße Verfahren umfaßt das Blockieren unspezifischer Bindestellen auf der bereits mit dem analytspezifischen Festphasenreaktanden belegten Festphase durch Inkubieren mit einem Alkylenoxid-modifizierten Bindemolekül, das als Blockierungssubstanz wirkt. Zeitdauer und Temperatur der Inkubation können innerhalb weiter Bereiche variiert werden, beispielhaft sind Inkubationstemperaturen von 4°C bis 40°C und Inkubationszeiten von 1 min bis 1 h.

Als Blockierungssubstanzen werden analytunspezifische bzw. inerte Biomoleküle verwendet; die an die Festphase bindefähig sind und nicht mit dem Nachweisverfahren interferieren, beispielsweise Proteine wie Albumine, unspezifische Antikörper oder Fragmente davon, oder Polysaccharide wie Dextrine etc. Die Bindung der Blockierungssubstanz an die Festphase erfolgt über hochaffine Bindepaare. Es wird eine Blockierungssubstanz verwendet,
die den zweiten Partner des Bindepaares umfaßt, z.B. ein biotinyliertes Protein, das einen oder mehrere Polyalkylenoxidreste enthält. Alternativ ist auch die Verwendung von Blockierungssubstanzen bevorzugt, bei denen ein oder mehrere Polyalkylenoxidreste direkt an den zweiten Partner des Bindepaares gekoppelt sind. Der zweite Partner des Bindepaares ist Biotin oder ein Biotinderivat.

Blockierungssubstanzen sind Konjugate der allgemeinen Strukturformeln (Ia) oder (Ib):

Pᵣ[-(AOₙ)T]ₘ (Ia)

Pᵣ-I-[-AOₙ)T]ₘ (Ib)

worin
- P: Biotin oder ein Biotinderivat ist,
- I: ein analytunspezifisches Biomolekül ist,
- r: eine Zahl von 1 bis 10 ist,
- AO: eine (C₂-C₃)-Alkylenoxidgruppe ist,
- n: eine Zahl von 5 bis 500 ist,
- T: eine Endgruppe vorzugsweise ausgewählt aus OH, C₁-C₄-Alkoxy und C₁-C₄-Acyl ist und
- m: eine Zahl von 1 bis 10 ist.

I ist vorzugsweise ein Polypeptid oder Saccharid. Bei Konjugaten der Formel (Ia) ist r vorzugsweise 1.

AO kann eine (C₂-C₃)-Alkylenoxidgruppe sein, d.h. eine Ethylenoxid- oder/und eine Propylenoxidgruppe. Vorzugsweise ist AO eine Ethylenoxidgruppe, doch auch Kombinationen von Ethylenoxid- und Propylenoxidgruppen sind geeignet. n ist vorzugsweise eine Zahl von 10 bis 250 und besonders bevorzugt 20 bis 200.

T ist eine Endgruppe (einschließlich des letzten O-Atoms der Polyalkylenoxidein-heiten), die mit weiteren Test- und Probenkomponenten kompatibel ist, d.h. nicht signifikant störende Reaktionen eingeht. Vorzugsweise ist T eine Hydroxylgruppe, C₁-C₄-Alkylethergruppe, insbesondere Methoxy, oder eine C₁-C₄-Acygruppe, z.B. eine Acetylgruppe. Bei Konjugaten der Strukturformel (Ia) ist m vorzugsweise 1.

Die Konjugate gemäß Strukturformel (Ia) und (Ib) werden vorzugsweise als Blockierungsreagenzien in Nachweisverfahren eingesetzt. Nach Immobilisierung auf einer Festphase sind sie vorzugsweise nicht mehr in der Lage, über die Komponente P eine hochaffine Bindung mit gelösten Biomolekülen in der Probe oder im Testreagenz einzugehen.

Ein weiterer Gegenstand der Erfindung ist eine Festphase mit einer Beschichtung, die ein oder mehrere Konjugate (Ib) und vorzugsweise einen analytspezifischen Festphasenreaktanden enthält. Die erfindungsgemäßen Konjugate können zur Verringerung der unspezifischen Bindung an eine Festphase in einem Verfahren zum Nachweis eines Analyten eingesetzt werden, beispielweise in einem immunologischen oder einem Nukleinsäure-Hybridisierungsverfahren. Ein weiterer Gegenstand des ersten Aspekts der vorliegenden Erfindung ist ein Reagenzienkit zum Nachweis eines Analyten, der neben anderen Testkomponenten ein erfindungsgemäßes Konjugat (Ib) oder eine erfindungsgemäße Festphase enthält.

In einer besonders bevorzugten Ausführungsform werden Biotin-Polyethylenglykolverbindungen verwendet, bei denen es sich um PEG-Ketten handelt, die an einem Kettenende mit einem Biotinrest funktionalisiert sind. Das andere Kettenende trägt vorzugsweise eine Hydroxyl- oder eine Methoxygruppe. Die Biotin-PEG-Konjugate werden auf eine Streptavidinfestphase nach oder gleichzeitig mit einem biotinylierten analytspezifischen Festphasenreaktanden, z.B. einem Antikörper, aufgebracht. Das Konjugat bindet auf den noch zugänglichen freien Biotinbindestellen der Streptavidinfestphase. Das nicht gebundene Biotin-PEG-Konjugat kann durch Waschen entfernt werden. Die resultierende Festphase kann in diesem Zustand getrocknet werden, ohne daß eine Beinträchtigung der Funktion auftritt. Die unspezifische Bindung einer mit einem erfindungsgemäßen Konjugat behandelten Oberfläche ist gegenüber einer unbehandelten Oberfläche oder gegenüber einer mit einer nicht-alkylenoxidmodifizierten Blockierungssubstanz behandelten Oberfläche stark vermindert. Ein weiterer Vorteil ist, daß die erfindungsgemäße Festphase auch nach dem Aufbringen des Festphasenreaktanden mit dem Blockierungskonjugat behandelt und damit mit den gewünschten Eigenschaften versehen werden kann. Bei Festphasen, welche begrenzte Testflächen und eine durchgehende Vorbeschichtung aufweisen, wird sowohl innerhalb der Testflächen als auch außerhalb dieser Testflächen (z.B. leere Streptavidinfestphase) eine deutliche Reduzierung der unspezifischen Bindung gefunden. Die Fähigkeit der Festphase zur Bindung des Analyten bleibt überraschenderweise unbeeinflußt.

Ein zweiter Anspekt der vorliegenden Erfindung ist ein heterogenes Verfahren zum Nachweis eines Analyten in einer Probe umfassend die Schritte:
(a) Bereitstellen einer Festphase, auf der ein Festphasenreaktand über ein hochaffines Bindepaar immobilisiert ist, wobei man einen universellen modifizierten Festphasenreaktanden verwendet, der mit einem Poly (C₂-C₃)-Alkylenoxid gekoppelt ist,
(b) Inkubieren der Probe mit der Festphase und einem Testreagenz und
(c) Nachweisen des Vorhandenseins oder/und der Menge des Analyten in der Probe.

Gemäß diesem zweiten Aspekt der Erfindung wird ein Polyalkylenoxid-modifizierter Festphasenreaktand auf der Festphase immobilisiert. Der modifizierte Festphasenreaktand ist ein universeller Festphasenreaktand, d.h. ein über ein hochaffines Bindepaar auf der Festphase immobilisierter Reaktand, der nicht spezifisch mit dem Analyten, sondern mit einem weiteren Festphasenreaktanden reagieren kann. Beispiele für universelle Festphasenreaktanden sind beispielsweise Streptavidin oder Anti-Hapten-Antikörper, die mit einem biotinylierten oder Hapten-konjugierten analytspezifischen weiteren Festphasenreaktanden reagieren können. Zusätzlich kann auch der analytspezifische Festphasenreaktand ein Polyalkylenoxid-modifizierter Festphasenreaktand sein.

Ein universeller modifizierter Festphasenreaktand kann beispielsweise ein Partner eines hochaffinen Bindepaares, oder ein Konjugat eines analytunspezifischen Biomoleküls mit einem Partner eines hochaffinen Bindepaares sein. Beispiele für universelle Festphasenreaktanden, die selbst den Partner eines hochaffinen Bindepaares darstellen, sind Polypeptide wie Streptavidin, Avidin, Hapten-spezifische Antikörper, Lectine und polymere Konjugate davon. Andererseits kann man auch ein Konjugat eines analytunspezifischen Biomoleküls mit einem Partner eines hochaffinen Bindepaares als universellen Festphasenreaktanden verwenden, beispielsweise ein inertes Polypeptid oder Polysaccharid gekoppelt mit Biotin, Biotinderivaten, Haptenen oder Zuckern.

Auch bei Verwendung eines analytspezifischen modifizierten Festphasenreaktanden geht es um ein Konjugat mit einem Partner eines hochaffinen Bindepaares. Beispiele solcher analytspezifischer modifizierter Festphasenrezeptoren sind analytspezifische Antikörper, Antigene, Nukleinsäuren, Nukleinsäurenanaloga und Lectine.

Weiterhin wird die Erfindung durch die nachfolgenden Beispiele erläutert.

### Beispiele

### 1. Synthese eines Biotin-Polyethylenglykol (PEG)-Konjugats (MW 3499)

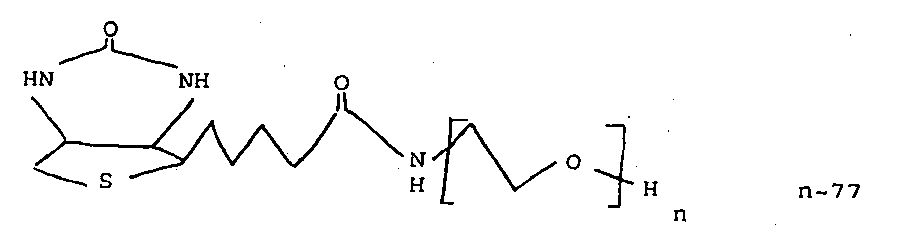

550 mg 1-Amino-PEG (Fa. Shearwater Polymers) wurden in 10 ml Dioxan gelöst. Zu dieser Lösung wurden 60 mg Triethylamin und anschließend 100 mg Biotin-OSu-Ester (Boehringer Mannheim) gegeben. Das Gemisch wurde 2,5 Stunden bei Raumtemperatur gerührt. Anschließend wurde das Produkt säulenchromatographisch gereinigt. Die Ausbeute betrug 30%.

### 2. Synthese eines Biotin-Methoxypolyethylenglykol-Konjugats (MW 5000)

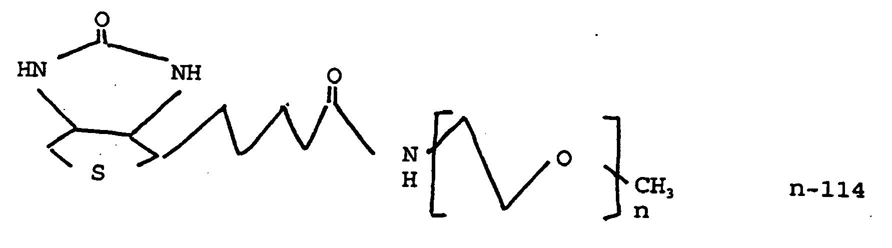

150 mg Aminomethoxy-PEG (Fa. Sigma) wurden in 100 ml Dioxan gelöst und anschließend mit 2 g Biotin-OSu-Ester, gelöst in 60 ml DMF, versetzt. Nach der Zugabe von 40 mg Triethylamin wurde 3 Stunden bei Raumtemperatur und weitere 3 Stunden bei 70°C gerührt. Die Lösungsmittel wurden anschließend abgezogen und das Produkt säulenchromatographisch gereinigt. Die Ausbeute betrug 57%.

### 3. Herstellung einer Biotin-PEG Festphase

Auf eine Strepatvidin Festphase (p-Styrol beschichtet mit an thermisch polymerisiertes Rinderserumalbumin (RSA) gebundenem Streptavidin) wurden mittels Microdosiertechnik biotinylierte, gegen TSH gerichtete Antikörper in Form von Flächen mit einem Durchmesser von ca. 0,1 mm aufgebracht. Diese Festphase wurde nach dem Aufbringen der Antikörperflächen mit Phosphatpuffer pH 7,5, der 50 µg/ml Bi-PEG enthält, nachbehandelt. Nach 2 minütiger Inkubation wurde nachgewaschen und getrocknet.

### 4. Untersuchung der unspezifischen Bindung an die Bi-PEG beschichtete Festphase

Die nach dem in Beispiel 3 beschriebenen Verfahren hergestellte Festphase wurde mit folgendem System bewertet: Nach Inkubation mit analytfreiem Probenmaterial (p24-freies Humanserum bzw. Enzymun®-TSH O-Standard) und anschließendem Waschschritt wurden die mit Humanserum inkubierten Festphasen mit digoxygeniliertem Nachweisreagenz (p24-Dig-Konjugat, sowie mit Anti-Human IgG-Antikörper-Dig-Konjugat) inkubiert. Die digoxygenilierten Reagenzien sind nicht spezifisch gegenüber dem Anti-TSH-Antikörper, d.h. sie enthalten keinen Analyten, stellen aber Marker für die Höhe der unspezifischen Bindung dar. Nach einem Waschschritt wurde die unspezifische Bindung durch einen fluoreszenzgefärbten, mit Anti-Dig-Antikörpern markierten Latex bestimmt. Die mittels fluoreszenzmikroskopischer Techniken erhaltenen Signale wurden mit optischer Bildauswertung quantifiziert und in Counts/sec. angegeben. Gemessen wurde die Fluoreszenzintensität innerhalb der Testflächen (mit biotinyliertem TSH-Antikörper) sowie außerhalb der Testflächen (Untergrund ohne Antikörperbeschichtung).

**Tabelle 1: Ergebnisse in der Testfläche (mit TSH-Anitkörpern) in Counts/sec**

| Festphase | Nachweisreagenz | | |
|---|---|---|---|
| | TSH 0-Standard | p24-Dig | < h-IgG >-Dig |
| ohne Bi-PEG | 65 | 1897 | 1612 |
| mit Bi-PEG | 31 | 740 | 1231 |

**Tabelle 2: Ergebnisse im Untergrund (Streptavidin-Festphase) in Counts/sec**

| Festphase | Nachweisreagenz | | |
|---|---|---|---|
| | TSH 0-Standard | p24-Dig | <h-IgG>Dig |
| ohne Bi-PEG | 51 | 766 | 654 |
| mit Bi-PEG | 17 | 136 | 140 |

In allen Fällen führte der Zusatz von Bi-PEG zu einer deutlichen Verminderung an unspezifischer Bindung auf der Festphase.

### 5. Synthese eines Streptavidin-Polyethylenglykol-Konjugats

Streptavidin und PEG-OSu wurden in Phosphatpuffer gelöst und in der jeweils gewünschten Stöchiometrie, vorzugsweise 1:1 bis 1:5 zusammengegeben. Nach 2 h Reaktion bei Raumtemperatur (?) wurde das Reaktionsgemisch gegen Phosphatpuffer mit 0,05% Natriumazid dialysiert und bei 4°C gelagert.

### 6. Herstellung einer universellen Streptavidin-PEG-Festphase

Ein Reaktionsgefäß wurde mit einer Lösung, die biotinyliertes Trägerprotein, (RSA-Biotin oder Thermo RSA-Biotin) in einer Konzentration von 100 µg/ml enthält, befüllt und 5 min bei Raumtemperatur inkubiert. Dann wurde die Lösung abgesaugt und das beschichtete Reaktionsgefäß mit Phosphatpuffer gespült und erneut abgesaugt.

Anschließend wurde Streptavidin-PEG in einer Konzentration von 50 µg/ml in Phosphatpuffer mit 1 % RSA zugegeben und 15 min inkubiert. Danach wurde die Lösung abgesaugt und durch Zugabe von Phosphatpuffer mit 1 % RSA und 2% Saccharose gewaschen. Nach erneutem Absaugen und Trocknen wurde die Festphase bei 4°C in luftdichter Verpackung gelagert.

### 7. Herstellung einer spezifischen Streptavidin-PEG-Festphase

Ein Reaktionsgefäß wurde mit einer Lösung, die biotinyliertes Trägerprotein in einer Konzentration von 100 µg/ml enthielt, befüllt und 5 min inkubiert. Die Lösung wurde abgesaugt, mit Phosphatpuffer gespült und erneut abgesaugt. Dann wurde Streptavidin-PEG (50µg/ml) in Phosphatpuffer mit 1 % RSA zugegeben und 15 min inkubiert. Die Lösung wurde abgesaugt, mit Phosphatpuffer gespült und erneut abgesaugt.

Dann wurde ein biotinylierter Antikörper, z.B. ein monoklonales Anti-TSH-Antikörper-Fab'₂-Fragment (5µg/ml) zugegeben und 15 min inkubiert. Die Lösung wurde abgesaugt, durch Zugabe von Phosphatpuffer mit 1 % RSA, 2% Saccharose gespült und erneut abgesaugt. Nach Trocknen wurde die Festphase bei 4°C in luftdichter Verpackung gelagert.

### 8.1 Bewertung von Streptavidin-PEG Festphasen

Ein Reaktionsgefäß mit der Festphase aus Beispiel 6 oder 7 wurde mit einer vorverdünnten analytfreien Probe (Pferdeserum 1:1 verdünnt mit Beladepuffer 50 mM Tris/HCl pH 7,5, 0,5% RSA, 0,05% Tween 20, 0,9% NaCl) 20 min bei Raumtemperatur inkubiert. Nach Waschen wurde 20 min in Gegenwart von Signalantikörper(1µg/ml monoklonaler Anti-TSH-Antikörper IgG-Digoxigenin-Konjugat in Beladepuffer) inkubiert und erneut gewaschen.

Nach Zugabe von Nachweisreagenz (0,01 % Lösung von Fluoro-Beads mit monoklonalem Anti-Digoxigenin-Antikörper-IgG beschichtet) wurde 20 min inkubiert, gewaschen und das Fluoreszenzsignal gemessen.

**Tabelle 3: Fluoreszenzleerwerte (arb. units) auf unterschiedlichen Festphasen**

| | unspezifische Festphase | spezifische Festphase |
|---|---|---|
| SA underivatisiert | 199 | 373 |
| SA-PEG (1:1) | 114 | 114 |
| SA-PEG (1:5) | (100) | (100) |

### 8.2 Unspezifische Bindung von Pufferbestandteilen

Ein Reaktionsgefäß mit der Festphase aus Beispiel 6 oder 7 wurde wie im Beispiel 8.2 beschrieben mit einer Pferdeserumprobe befüllt und gewaschen. Dann wurden 0,2 µg/ml p24-Digoxigenin in Beladepuffer zugegeben, 20 min inkubiert und gewaschen. Dann wurde Nachweisreagenz (vgl. 8.1) zugegeben, erneut 20 min inkubiert, gewaschen und das Fluoreszenzsignal gemessen. Die Ergebnisse sind in Tabelle 4 dargestellt.

**Tabelle 4: unspezifische Bindung von p24-Digoxigenin (arb. units) auf unterschiedlichen Festphasen**

| | unspezifische Festphase | spezifische Festphase |
|---|---|---|
| SA underivatisiert | 691 | (>1500) |
| SA-PEG (1:1 | 260 | 660 |
| SA-PEG (1:5) | 124 | 365 |

### 8.3 Unspezifische Bindung von humanen IgG-Antikörpern

Ein Reaktionsgefäß mit den in Beispiel 6 und 7 hergestellten Festphasen wurde wie in 8.1 beschrieben, mit einer Probe befüllt und gewaschen. Die Probe war Humanserum, 1:19 mit Beladepuffer verdünnt.

Dann wurden 1,0 µg/ml monoklonaler Anti-human-IgG-Antikörper-Digoxigenin-Konjugat in Beladepuffer zugegeben und gewaschen. Anschließend wurde das Nachweisreagenz zugegeben, 20 min inkubiert, erneut gewaschen und das Fluoreszenzsignal gemessen. Die Ergebnisse sind in der nachfolgenden Tabelle 5 dargestellt.

**Tabelle 5: unspezifische Bindung von Humanantikörpern (arb. units) auf unterschiedlichen Festphasen**

| | unspezifische Festphase | spezifische Festphase |
|---|---|---|
| SA underivatisiert | 2549 | (>30001 |
| SA-PEG (1:1) | 944 | 1749 |
| SA-PEG (1:5) | 515 | 834 |

### 9. Herstellung von Antikörper-PEG-Konjugaten

Die Herstellung von PEG-Antikörper-Konjugaten erfolgte wie in Beispiel 5 beschrieben, außer daß an Stelle von Streptavidin ein biotinylierter Antikörper verwendet wurde.

### 10. Herstellung von mit PEG-Antikörper-Konjugaten beschichteten Festphasen

Ein Reaktionsgefäß wurde mit einer Lösung, die mit 100 µg/ml biotinyliertes Trägerprotein (RSA-Biotin oder tRSA-Biotin) enthält, 5 min inkubiert. Dann wurde die Lösung abgesaugt, mit Phosphatpuffer gespült und erneut abgesaugt.

Anschließend wurden 50 µg/ml Streptavidin in Phosphatpuffer mit 1 % RSA zugegeben und 15 min inkubiert. Diese Lösung wurde abgesaugt, mit Phosphatpuffer gespült und erneut abgesaugt. Anschließend wurden 5 µg/ml biotinylierter IgG-Antikörper, z.B. ein monoklonales Anti-TSH-Fab₂-Antikörperfragment, zugegeben und 15 min inkubiert. Die Lösung wurde abgesaugt und ein Spülschritt mit Phosphatpuffer, 1 % RSA, 2% Saccharose durchgeführt. Nach erneutem Absaugen wurde das Reaktionsgefäß getrocknet und bei 4°C in luftdichter Verpackung gelagert.

### 11. Bewertung

### 11.1 Leerwert

Die Bestimmung eines Leerwerts der in Beispiel 10 hergestellten Festphase erfolgte wie in Beispiel 8.1 beschrieben. Die Ergebnisse sind in Tabelle 6 dargestellt.

**Tabelle 6: Fluoreszenzleerwerte (arb. units) auf unterschiedlichen Festphasen**

| | Signale (arb. units) |
|---|---|
| AK underivatisiert | 270 |
| AK-PEG (1:1) | 94 |
| AK-PEG (1:5) | 57 |

### 11.2 Unspezifische Bindung von Pufferbestandteilen

Die unspezifische Bindung von Pufferbestandteilen an die in Beispiel 10 hergestellte Festphase wurde wie unter Beispiel 8.2 beschrieben bestimmt. Die Ergebnisse sind in Tabelle 7 dargestellt.

**Tabelle 7: unspezifische Bindung von p24-Digoxigenin (arb. units) auf unterschiedlichen Festphasen**

| | spezifische Festphase |
|---|---|
| AK underivatisiert | 26658 |
| AK-PEG (1:1) | 23519 |
| AK-PEG (1:5) | 7998 |

### 11.3 Bestimmung der unspezifischen Bindung von Human-Antikörpern (IgG)

Die Bestimmung der unspezifischen Bindung von humanen IgG-Antikörpern an die in Beispiel 10 hergestellte Festphase erfolgte wie unter Beispiel 8.3 beschrieben. Die Ergebnisse sind in Tabelle 8 dargestellt.

**Tabelle 8: unspezifische Bindung von Humanantikörpern (arb. units) auf unterschiedlichen Festphasen**

| | spezifische Festphase |
|---|---|
| AK underivatisiert | 11379 |
| AK-PEG (1:1) | 10475 |
| AK-PEG (1:5) | 4446 |

### Beispiel 12 Durchführung eines <HIVI> Tests und Testergebnisse mit Negativproben

Auf einen Polystyrolträger wurde auf eine Testfläche von ca. 100 µm Durchmesser ein Antigen aufgebracht, welches das gp41 des HIV-I-Virus repräsentiert. Auf die Testfläche wurden 30 µl mit Probenpuffer vorverdünnte Probe pipettiert und 20 Minuten unter Schütteln bei Raumtemperatur inkubiert. Nach Absaugen der Probe und Waschen des Testfeldes mit Waschpuffer wurden 30 µl Reagenzlösung mit einem Dig-markierten gp41-repräsentierenden HIV I-Antigen pipettiert und wiederum 20 Minuten unter Schütteln bei Raumtemperatur inkubiert. Nach Absaugen der Reagenzlösung und Waschen des Testfeldes mit Waschpuffer werden 30 µl Nachweisreagenz auf das Testfeld pipettiert. Als Nachweisreagenz dienen 100 nm große, fluoreszenzgefärbte Latexpartikel, die kovalent mit einem Anti-Dig-Antikörper beschichtet sind.

Dieses Nachweisreagenz wurde wiederum 20 Minuten unter Schütteln bei Raumtemperatur inkubiert, anschließend abgesaugt, gewaschen und trockengesaugt. Das Testfeld wurde mit einem He-Ne-Laser mit 633 nm Wellenlänge bestrahlt und die Fluoreszenz bei 670 nm Wellenlänge mit einer CCD-Kamera vermessen.

Folgende testspezifischen Reagenzien wurden verwendet:

| | |
|---|---|
| Festphasenantigen: | Polyhapten aus gp41-Peptid |
| Nachweisantigen: | Polyhapten aus pg41-Peptid, Dig-markiert. |

Folgende Meßwerte (Counts) wurden gemessen:

| Probe | Untergrund" [Counts] | Signal Testfeld [Counts] | Signal Testfeld - Untergrund | Cut-off- Index** |
|---|---|---|---|---|
| Negativkontrolle | 148 | 148 | 0 | 0,0 |
| Positiv Probe 1 | 178 | 26435 | 26257 | 88,1 |
| Positivprobe 2 | 172 | 22908 | 22376 | 76,8 |
| Negativprobe 1 | 101 | 101 | 0 | 0,0 |
| Negativprobe 2 | 103 | 103 | 0 | 0,0 |
| Negativprobe 3 | 93 | 93 | 0 | 0,0 |
| Negativprobe 4 | 98 | 98 | 0 | 0.0 |
| Negativprobe 5 (S441) | 86 | 4401 | 4315 | 14,6 |
| Negativprobe 6 (S480) | 137 | 2690 | 2553 | 8,6 |
| Negativprobe 7 (S486) | 107 | 3833 | 3726 | 12,6 |
| Negativprobe 8 (S520) | 116 | 4331 | 4215 | 14,2 |

| | | | | |
|---|---|---|---|---|
| * Untergrund entspricht Signal neben den Testfeldern ** Cut-off-Index = Signal_{Probe}-Signal_{Untergrund}/2 × Signal_{Negativkontrolle} Cut-off-Index < 1 = negativ | | | | |

Die obige Tabelle spiegelt einen Ausschnitt aus einer Spezifitätsstudie wieder. In dieser Studie wurden ca. 240 <HIV I> negative Proben vermessen. Der Großteil der Proben (z.B. Negativproben 1-4) zeigte keine Reaktion auf den Testfeldern und war somit eindeutig negativ. Allerdings wurden 4 Proben (Negativproben 5-8) gefunden, die eine starke unspezifische Reaktion auf den Testfeldern zeigten und somit als falsch positiv detektiert wurden.

### Beispiel 13 Verbesserung der Spezifität durch PEG-derivatisiertes Festphasenantigen

In diesem Versuch wurde ein <HIV I> Test analog Beispiel 12 durchgeführt. Im Unterschied dazu wurden auf dem identischen Testträger neben dem HIV I-Antigen zusätzlich ein identisches Antigen aufgebracht, welches im Stöchiometrieverhältnis von 1:1 mit PEG 500 derivatisiert wurde.

Folgende Meßwerte wurden erhalten:

| Probe | Untergrund* [Counts] | Polyhapten-gp41-Peptid | | Polyhapten-gp41-Peptid-PEG | |
|---|---|---|---|---|---|
| | | Counts** | COI*** | Counts** | COI*** |
| Negativ-Kontr. | 52 | 0 | 0.0 | 31 | 0.3 |
| Positiv-Kontr. | 63 | 286 | 2.0 | 8383 | 80 |
| Positivprobe 1 | 212 | 11752 | 111 | 6227 | 57.8 |
| Positivprobe 2 | 84 | 1632 | 14.9 | 3762 | 35.3 |
| S441 | 50 | 1061 | 9.7 | 79 | 0.3 |
| S480 | 53 | 871 | 7.9 | 102 | 0.5 |
| S486 | 44 | 1041 | 9.6 | 98 | 0.5 |
| S520 | 44 | 1260 | 11.7 | 84 | 0.4 |

| | | | | | |
|---|---|---|---|---|---|
| * Untergrund entspricht Signal neben den Testfeldern **Signal_{Probe}-Signal_{Untergrund} *** COI = Cut-off-Index = Signal_{Probe}-Signal_{Untergrund}/2 _{×} Signal_{Negativkontrolle} Cut-off-Index < 1 = negativ | | | | | |

Dieses Ergebnis zeigt, daß die unspezifische Bindung der Störproben in den <HIV I>-Testfläche durch Verwendung des neuen PEG-derivatisierten Antigens drastisch reduziert wird, so daß alle 4 Störproben negativ werden. Überraschenderweise kann die PEG-Derivatisierung sogar zu einer starken Erhöhung des Signals von Positivproben führen (siehe Positivkontrolle und Positivprobe 1).

### 14. Nachweis von HBs-Antigen

Auf einen Polystyrolträger wurde auf eine Testfläche von ca. 100 µm Durchmesser ein monoklonaler Antikörper gegen HBs-Antigen aufgebracht. Auf eine weitere Testfläche wurde derselbe Antikörper in Form eines PEG-Konjugats (Herstellung Beispiel 9) aufgebracht. Auf die Testfläche wurden dann 30 µl mit Probenpuffer vorverdünnte Probe pipettiert und 20 min unter Schütteln in Raumtemperatur inkubiert. Nach Absaugen der Probe und Waschen der Testfläche mit Waschpuffer werden 30 µl Reagenzlösung mit Digoxigenin-markiertem Anti-HBsAg-Antikörper zupipettiert und wiederum 20 min unter Schütteln bei Raumtemperatur inkubiert. Nach Absaugen der Lösung und Waschen der Testfläche mit Waschpuffer wurden 30 µl Nachweisreagenz (Beispiel 8.3) auf die Testfläche pipettiert.

Der Nachweis erfolgte wie in Beispiel 8.1 beschrieben.

Untersucht wurden ein positiver Standard, ein negativer Standard sowie fünf Negativseren, die kein HBsAg enthalten, aber im Test dennoch signifikant positive Signale liefern, die auf analytunspezifische Wechselwirkungen mit der Festphase zurückzuführen waren. In der Tabelle 9 sind die Ergebnisse dieser Versuche aufgelistet. Es ist klar zu erkennen, daß die unspezifischen Bindungen mit PEG-derivatisierten Antikörper sehr viel niedriger ausfallen, als bei unbehandelten Antikörpern.

**Tabelle 9**

| | **Meßsignal** | |
|---|---|---|
| **Probe** | **MAK<HBs>** | **MAK<HBs>PEG** |
| Pos. Standard | 1080 | 960 |
| Neg. Standard | 1,3 | 1,7 |
| Negativserum 1 | 16 | 3,4 |
| Negativserum 2 | 28 | 12 |
| Negativserum 3 | 15 | 1,3 |
| Negativserum 4 | 11,5 | 2 |
| Negativserum 5 | 18 | 9 |

## Patentansprüche

1. Heterogenes Verfahren zum Nachweis eines Analyten in einer Probe, umfassend die Schritte:
(a) Bereitstellen einer Festphase umfassend in immobilisierter Form einen analytspezifischen Festphasenreaktanden und ein analytunspezifisches Biomolekül, das mit einem Poly (C₂-C₃)-Alkylenoxid gekoppelt ist, wobei die Festphase mit einem ersten Partner eines hochaffinen Bindepaares belegt ist, an die ein Konjugat des analytspezifischen Festphasenreaktanden mit dem zweiten Partner des Bindepaares immobilisiert ist und wobei als analytunspezifisches Biomolekül eine Blockierungssubstanz verwendet wird, die den zweiten Partner des Bindepaares umfaßt und ausgewählt ist aus Konjugaten der allgemeinen Strukturformel (Ia) oder (Ib):
Pᵣ[- (AOₙ)T]ₘ (Ia)
Pᵣ-I-[-(AOₙ)T]ₘ (Ib)
worin
P Biotin oder ein Biotinderivat ist,
I ein analytunspezifisches Biomolekül ist,
r eine Zahl von 1 bis 10 ist,
AO eine (C₂-C₃)-Alkylenoxidgruppe ist,
n eine Zahl von 5 bis 500 ist,
T eine Endgruppe vorzugsweise ausgewählt aus OH, C₁-C₄-Alkoxy und C₁-C₄-Acyl ist und
m eine Zahl von 1 bis 10 ist,
(b) Inkubieren der Probe mit der Festphase und einem Testreagenz und
(c) Nachweisen des Vorhandenseins oder/und der Menge des Analyten in der Probe.

2. Verfahren nach Anspruch 1,
**dadurch gekennzeichnet,**
**daß** man eine Festphase verwendet, die mindestens eine begrenzte Testfläche aufweist.

3. Verfahren nach Anspruch 1 oder 2,
**dadurch gekennzeichnet,**
**daß** man eine Blockierungssubstanz verwendet, bei der ein oder mehrere Polyalkylenoxidreste direkt an den zweiten Partner des Bindepaares gekoppelt sind.

4. Konjugate der allgemeinen Strukturformel (Ib):
Pᵣ-I-[-(AOₙ)T]ₘ (Ib)
worin
P Biotin oder ein Biotinderivat ist,
I ein analytunspezifisches Biomolekül ist,
r eine Zahl von 1 bis 10 ist,
AO eine (C₂-C₃)-Alkylenoxidgruppe ist,
n eine Zahl von 5 bis 500 ist,
T eine Endgruppe vorzugsweise ausgewählt aus OH, C₁-C₄-Alkoxy und C₁-C₄-Acyl ist und
m eine Zahl von 1 bis 10 ist.

5. Festphase mit einer Beschichtung, die ein Konjugat nach Anspruch 4 enthält.

6. Verwendung eines Konjugats nach Anspruch 4 zur Verringerung der unspezifischen Bindung an eine Festphase in einem heterogenen Verfahren zum Nachweis eines Analyten.

7. Reagenzienkit zum Nachweis eines Analyten, der neben anderen Testkomponenten ein Konjugat nach Anspruch 4 oder eine Festphase nach Anspruch 5 enthält.

8. Heterogenes Verfahren zum Nachweis eines Analyten in einer Probe umfassend die Schritte:
(a) Bereitstellen einer Festphase, auf der ein Festphasenreaktand über ein hochaffines Bindepaar immobilisiert ist, wobei man einen universellen modifizierten Festphasenreaktanden verwendet, der mit einem Poly (C₂-C₃)-Alkylenoxid gekoppelt ist,
(b) Inkubieren der Probe mit der Festphase und einem Testreagenz und
(c) Nachweisen des Vorhandenseins oder/und der Menge des Analyten in der Probe.

## Claims

1. A heterogeneous method for the detection of an analyte in a sample comprising the steps:
(a) preparing a solid phase comprising, in an immobilised form, an analyte-specific solid phase reactant and an analyte-nonspecific biomolecule which is coupled to a poly (C₂-C₃)-alkylene oxide, wherein the solid phase is coated with a first partner of a high-affinity binding pair, on which a conjugate of the analyte-specific solid phase reactant is immobilised with the second partner of the binding pair, and wherein a blocking substance is used as the analyte-nonspecific biomolecule which comprises the second partner of the binding pair and is selected from conjugates of the general structural formula (Ia) or (Ib):
Pᵣ[-(AOₙ)T]ₘ (Ia)
Pᵣ-I-[-(AOₙ)T]ₘ (Ib)
in which
P is biotin or a biotin derivative,
I is an analyte-nonspecific biomolecule,
r is a number from 1 to 10,
AO is a (C₂-C₃)-alkylene oxide group,
n is a number from 5 to 500, .
T is an end group preferably selected from OH, C₁-C₄-alkoxy and C₁-C₄ acyl and
m is a number from 1 to 10,
(b) incubating the sample with the solid phase and a test reagent and
(c) detecting the presence and/or the amount of the analyte in the sample.

2. A method according to Claim 1, **characterised in that** a solid phase is used which has at least one defined test area.

3. A method according to Claim 1 or 2, **characterised in that** a blocking substance is used, in which one or more polyalkylene oxide residues are directly coupled to the second partner of the binding pair.

4. Conjugates of the general structural formula (Ib):
Pᵣ-I-[-(AOₙ)T]ₘ (Ib)
in which
P is biotin or a biotin derivative,
I is an analyte-nonspecific biomolecule,
r is a number from 1 to 10,
AO is a (C₂-C₃)-alkylene oxide group,
n is a number from 5 to 500,
T is an end group preferably selected from OH, C₁-C₄-alkoxy and C₁-C₄ acyl and
m is a number from 1 to 10.

5. A solid phase with a coating which contains a conjugate according to Claim 4.

6. Use of a conjugate according to Claim 4 for reducing the non-specific binding to a solid phase in a heterogeneous method for the detection of an analyte.

7. A reagent kit for the detection of an analyte, which, in addition to other test components, contains a conjugate according to Claim 4 or a solid phase according to Claim 5.

8. A heterogeneous method for the detection of an analyte in a sample comprising the steps of:
(a) preparing a solid phase, on which a solid phase reactant is immobilised via a high-affinity binding pair, wherein a universally modified solid phase reactant is used which is coupled to a poly (C₂-C₃)-alkylene oxide,
(b) incubating the sample with the solid phase and a test reagent and
(c) detecting the presence and/or the amount of the analyte in the sample.

## Revendications

1. Procédé de détection hétérogène d'un analyte dans un échantillon, comprenant les étapes consistant à :
(a) se munir d'une phase solide comprenant sous forme immobilisée, des réactifs en phase solide spécifiques à l'analyte et une biomolécule non spécifique à l'analyte, qui est couplée à un polyoxyde d'alkylène (en C₂ à C₃), la phase solide étant dotée d'un premier partenaire d'une paire de liaison hautement affine, sur laquelle un conjugué du réactif en phase solide spécifique à l'analyte est immobilisé avec le deuxième partenaire de la paire de liaison et en utilisant comme biomolécule non spécifique à l'analyte, une substance de blocage qui comprend le deuxième partenaire de la paire de liaison et est choisie parmi des conjugués de formules structurelles générales (Ia) ou (Ib) :
Pr[-(AOₙ)T]ₘ (Ia)
Pr-I-[-(AOₙ)T]ₘ (Ib)
dans lesquelles
P désigne la biotine ou un dérivé de biotine,
I désigne une biomolécule non spécifique à l'analyte
r est un nombre de 1 à 10 et
AO est un groupe d'oxyde d'alkylène (en C₂ à C₃),
n est un nombre de 5 à 500,
T désigne un groupe terminal choisi de préférence parmi OH, un groupe alcoxy en C₁ à C₄ et un groupe acyle en C₁ à C₄, et
m est un nombre de 1 à 10,
(b) incuber l'échantillon avec la phase solide et un réactif de test, et
(c) détecter la présence et/ou la quantité d'analyte dans l'échantillon.

2. Procédé selon la revendication 1,
**caractérisé en ce**
**que** l'on utilise une phase solide qui présente au moins une surface de test limitée.

3. Procédé selon la revendication 1 ou 2,
**caractérisé en ce**
**que** l'on utilise une substance de blocage dans laquelle un ou plusieurs résidus poly(oxyde d'alkylène) sont couplés directement au deuxième partenaire de la paire de liaison.

4. Conjugué de formule structurelle générale (Ib) :
Pr-I-[-(AOn)T], (Ib)
dans laquelle
P désigne la biotine ou un dérivé de biotine,
I désigne une biomolécule non spécifique à l'analyte
r est un nombre de 1 à 10 et
AO est un groupe d'oxyde d'alkylène (en C₂ à C₃),
n est un nombre de 5 à 500,
T désigne un groupe terminal choisi de préférence parmi OH, un groupe alcoxy en C₁ à C₄ et un groupe acyle en C₁ à C₄, et
m est un nombre de 1 à 10.

5. Phase solide comportant un revêtement qui contient un conjugué selon la revendication 4.

6. Utilisation d'un conjugué selon la revendication 4, pour diminuer la liaison non spécifique sur une phase solide dans un procédé de détection hétérogène d'un analyte.

7. Kit de réactifs pour la détection d'un analyte qui contient, parallèlement à d'autres composants de test, un conjugué selon la revendication 4 ou une phase solide selon la revendication 5.

8. Procédé de détection hétérogène d'un analyte dans un échantillon, comprenant les étapes consistant à :
(a) se munir d'une phase solide sur laquelle est immobilisé un réactif en phase solide par une paire de liaison hautement affine, en utilisant un réactif en phase solide universel modifié qui est couplé à un polyoxyde d'alkylène (en C₂ à C₃),
(b) incuber l'échantillon avec la phase solide et un réactif de test, et
(c) détecter la présence et/ou la quantité d'analyte dans l'échantillon.
